# EUROPEAN PATENT APPLICATION

(11) **EP 4 223 528 A1**
(43) Date of publication of application: **09.08.2023**
(21) Application number: 21783327.6
(22) Date of filing: 30.08.2021
(51) Int. Cl.: B33Y 70/00, A61L 27/24, A61L 27/38, A61L 27/52

(54) **COLLAGEN INK FOR 3D PRINTING**

(30) Priority: 02.10.2020 ES 202030996
(71) Applicant: Viscofan, S.A., 31192 Tajonar (Navarra) (ES)
(72) Inventor: ZÚÑIGA ARRARÁS, Teresa, 69469 WEINHEIM (DE); GUEMBE LAPUENTE, Amaia, 31192 TAJONAR (Navarra) (ES); RECALDE IRURZUN, José, Ignacio, 31192 TAJONAR (Navarra) (ES); IZCO ZARATIEGUI, Jesús, María, 31192 TAJONAR (Navarra) (ES)
(74) Representative: Herrero & Asociados, S.L.
(86) International application number: PCT/ES2021/070627
(87) International publication number: WO 2022/069772

(57) **Abstract**

The present invention relates to a collagen ink for 3D printing. The present invention also relates to the method of obtaining the ink and the uses given to it.

## Description

The present invention relates to a collagen ink for 3D printing. The present invention also relates to the method of obtaining the ink and the uses given to it.

### Background of the invention

3D printing is a widely used technique in the area of biology and medicine where biological structures are obtained to provide a service to regenerative medicine and biological studies.

The inks to be used in 3D printing, must have adequate physical properties in terms of, for example, viscosity, elasticity, porosity and firmness, but they must also be compatible with different applications such as tissue development, so they must possess adequate biological characteristics for it. The ink must be fluid enough to facilitate extrusion through the printer nozzle and the printed structure must remain intact over time at physiological pH.

Examples of materials used in inks for biological applications are alginates, fibrins and collagen.

Collagen is one of the most abundant proteins in nature and is responsible for maintaining the structural integrity of tissues. Collagen is the material most used in cell applications since it is the most abundant component in the extracellular matrix; this material is used for example as a support matrix to help cell growth. Collagen fibers have certain amino acid sequences, which allow cells to adhere and proliferate. Collagen matrices are very useful and have produced several important biological advances; however, an optimal 100% collagen matrix has not been developed. Compositions consisting only of collagen are made from soluble collagen and do not usually have sufficient consistency and firmness to be used as inks that maintain the integrity of the printed structure without the aid of crosslinking agents or do not have a native configuration.

In the case of collagen, so that it has sufficient viscosity and firmness to be used as an ink, it can be presented in the form of a hydrogel. An example of this is found in the patent with publication number CN106581753 which describes a biological hydrogel for the 3D printing of a matrix for the growth of dermal tissue. The hydrogel comprises 1% -15% nanocrystalline cellulose, 65% -98% collagen and 0.01% -20% cross-linking agent. This type of hydrogels have the appropriate viscosity to be used as inks, but the construct that is obtained with it loses its integrity if a cross-linking agent is not added to the collagen so that they can perform their function.

To give collagen the necessary characteristics to be used as a 3D printing ink in biological applications, the strategy of functionalizing collagen has been used. Patent with publication number CN106237383 is an example which describes functionalized collagen microfilaments with a growth factor that promotes cell growth and differentiation. In this case, a classification and previous selection of the fibers is also made, obtaining fibers of very small size, adjusting said size so that it is less than the diameter of the needle of the extrusion head (for example, a 27G needle has an internal diameter of 210µm), it is based on a soluble collagen extracted with surfactants such as SDS and triton and it is also modified with another molecule to obtain optimal results. In addition, no rheological data or the firmness of the ink achieved, or its printability or the integrity of the constructs obtained, is indicated. In said patent fibers of 100, 75, 38 and reaching up to 25 µm are selected.

Other strategies that have been used for the production of collagen matrices for 3D printing are cryogenic printing that allows the deposition of a low viscosity collagen solution at sub-zero temperatures and performing crosslinking in situ.

The extraction and purification methods currently applied to process collagen markedly reduce its crosslinking density with consequent impact on relevant properties and biological response.

Therefore, based on the state of the art, it is necessary to develop inks based on fibrillar collagen, which has not been denatured and which has sufficient consistency and firmness to be used as inks in 3D printing without help of crosslinking agents.

### Description of the invention

Collagen is the most abundant protein within the extracellular matrix of connective tissues such as skin, bone, cartilage and tendon of mammals and comprises more than 90% of their dry weight.

The essential unit of collagen is made up of three polypeptide chains that appear intertwined forming a triple helix, constituting a macromolecular unit called tropocollagen. Tropocollagen molecules group together to form collagen fibers.

In the present invention, a collagen ink for 3D printing has been developed with a suitable viscosity to be used in 3D printers and keeping the collagen in its native state. The ink of the invention is characterized by being a dispersion of native collagen fibers in an acidic aqueous medium.

The acidic medium allows the collagen to absorb water and results in an extrudable fluid.

Therefore, a first aspect of the invention relates to a collagen ink for 3D printing comprising a dispersion of native collagen fibers in an acid medium at a concentration by weight of between 0.1% and 10% with a pH between 0.5 and 5, which has a viscosity between 10 centipoise (cP) and 50 million centipoise (McP) (Brookfield method at a temperature between 18°C and 22°C) and wherein the fiber dispersion comprises fibers whose length, measured at a pH between 1 and 2, is in a range from 1 µm to 2500 µm and its diameter is in a range from 0.1 µm to 180 µm.

The fibers in the ink of the invention have a wide range of lengths and diameters and no sorting is necessary to obtain and select the small size fibers of the ink. The ink is printable even though it can have a high percentage of its mass with large fibers, with fibers greater than 1 mm in length and with diameters that can be greater than the internal diameter of the printer's extrusion head, for example, 22G = 152um.

The collagen fiber of the invention maintains the triple helix molecular structure without being denatured. The fiber can absorb water and swell, with diameters that are mainly between 5µm and 35µm, while said fibers would be absent in case of denaturation and hydrolysis.

As we have mentioned, the collagen molecule is an elongated molecule made up of three polypeptide chains twisted together, constituting a triple helix stabilized by hydrogen bridge bonding or interactions, this is the structure of the native collagen. When collagen is denatured, for example by heating it in the presence of water or heating it to extreme pH values, these chains separate and dissolve, forming a gelatin. The formation of gelatin is due to the breaking of the hydrogen bonds that stabilize the collagen triple helix, the process of transformation of collagen into gelatin is considered a typical denaturation process. In the present dispersion of collagen fibers, there is no gelatin or its presence is not significant.

In the present description, "native collagen" is understood to mean collagen that has not been completely or significantly denatured, hydrolyzed or gelatinized.

The ink of the invention is presented in the form of a dispersion in an acid medium of native fibrillar collagen of optimized size, obtained from a biological source of collagen, normally consisting of large bundles and / or fibrillar aggregates subjected to treatments that, preserving the native structure, trigger the disintegration into fibers of smaller diameter and length which allow formulating fluids with the rheological characteristics suitable for a correct 3D printing.

The structures that are obtained with the ink of the invention by 3D printing are stable and resistant structures of native, non-soluble fibrous collagen, without the need to use crosslinking agents.

It is important that the ink includes native collagen since in the natural environment collagen is not only a structural and support protein, but it specifically interacts with other biomolecules, mediates cell adhesion and guides cell functions. As long as the ink of the invention preserves its native structure, when the ink is used in prints for biomedical applications, it will be able to maintain its characteristics in relation to its physiological functions.

Likewise, the invention relates to any hydrogel comprising the ink described in the first aspect of the invention and in the embodiments described below.

A second aspect of the invention is the method of obtaining collagen ink that comprises the steps of:
a) washing, and chopping a collagen-containing tissue;
b) chemically macerating the chopped tissue;
c) washing the product obtained from step b) with water;
d) adjusting the pH of the side product obtained in c), to values between 0.5 and 5 to swell the side product of step c):
e) mechanically mincing the product of step d);
f) dispersing in water to a concentration by weight preferably between 0.1% and 10%.

The collagen matrix has low immunogenicity, good biocompatibility and biodegradability, specifically interacts with other biomolecules, contains specific sequences that mediate the regulation of cell morphology, adhesion, migration and differentiation. The ink of the invention has the appropriate viscosity to pass through the 3D printing nozzle.

Therefore, the third aspect of the invention relates to the use of the ink of the invention in 3D printing. Also described in the present invention is the method of printing with the ink described above and in any of its embodiments.

The fourth aspect of the invention relates to the structure comprising the ink of the invention. One of the advantages of this structure is that it is highly biocompatible and resorbable.

### Brief description of the drawings

Figure 1 shows a mesh printed with the ink of the invention as a biodegradable scaffold for cell culture.
Figure 2 shows the culture of the meshes with murine embryonic cell fibroblasts in the mesh of Figure 1 after two days of culture obtained on day 2 (10X).
Figure 3 shows the culture on day 5.
Figure 4 shows the culture on the sixth day of development.
Figure 5 shows the stress sweep plot of samples 531-010, 531-020.
Figure 6 shows the stress sweep plot of samples 431-010, 431-020.
Figure 7 shows the construct printed with the ink of the invention.
Figures 8A-8F show different constructions made with the ink. Figures 8B, 431-010 and 8F correspond to constructs A, C and E after neutralizing and adjusting to physiological pH (pH 7.4).
Figure 9 and 9B shows optical microscopy images of individual particles of the ink of the invention 531-010 diluted to 0.1% and stained with Sirius Red..
Figure 10 shows the element printed with the ink of Example 8.

### Description of a preferred embodiment

As mentioned above, the first aspect of the invention relates to a collagen ink for 3D printing that comprises a dispersion of native collagen fibers in an acid medium at a concentration between 0.1% and 10% with a pH between 0.5 and 5, which has a viscosity between 10 cP and 50 McP (according to the Brookfield method described in Example 3 and at a temperature between 18 ° C and 22 ° C,) and wherein the fiber dispersion comprises fibers whose length, measured at a pH between 1 and 2, is in a range of 1µm to 2500µm and its diameter in a range of 0.1 µm to 180µm.

Preferably 90% of the mass of collagen fibers in the dispersion comprises fibers whose length is in a range from 50 µm to 2500 µm.

Preferably 75% of the mass of collagen fibers in the dispersion comprises fibers whose length is in a range of 50 µm to 1000 µm and 50% between 100 µm and 500 µm.

Preferably 80% of the mass of collagen fibers comprises fibers whose diameter is between 5 µm and 35 µm.

Preferably the concentration is between 1% and 5%.

Preferably, the pH is between pH 1 and 3.

Preferably the viscosity is between 2 McP and 15 McP.

A characteristic of the collagen matrices of the invention is its integrity after neutralizing it to physiological pH.

A characteristic of the collagen matrices of the invention is its integrity after neutralizing it to physiological pH. Among the possible applications of the ink of the invention is the printing of matrices for cell and tissue culture. In this case, it is very advantageous for the collagen to be native since in this way it more closely resembles the original structure of collagen, replicating with greater similarity the original environment in which cell proliferation takes place in the body. Surprisingly, the construct or matrix obtained is rigid enough to remain intact and firm over time at physiological pH. Preferably, the ink used in these applications comprises a dispersion of native collagen fibers in an acid medium at a concentration between 2% and 5% with a pH between 1 and 3.

A preferred use of the ink described in the previous paragraph is the construction of matrices for the culture of cells and tissues.

As has been said, the second aspect of the invention relates to the method of obtaining the ink of the invention from a collagen-containing tissue that comprises the steps of:
a) washing, and chopping a collagen-containing tissue;
b) chemically macerating the chopped tissue;
c) washing the product obtained from step b) with water;
d) adjusting the pH of the side product obtained in c), to values between 0.5 and 5 to swell the side product of step c):
e) mechanically mincing the product of step d);
f) dispersing in water to a concentration preferably between 0.1% and 10%.

Preferably the collagen-containing tissue is connective tissue. The connective tissue is preferably selected from: dermis, bone, tendon, cartilage, intestine. Preferably the connective tissue is dermis tissue and more preferably the collagen is extracted from the layer called corium.

As for the origin of connective tissue, it can come from any animal source. In a particular embodiment, the connective tissue is of bovine, ovine, porcine, avian, fish origin or a mixture thereof. Preferably, the connective tissue is of bovine origin and of an animal of more than one year of age. Preferably between 1 and 3 years.

More preferably before step a) when the connective tissue is the dermis, it is depilated and bleached. A bleaching stage with weak oxidizing agents is preferably carried out in addition to washing stage a). An example of a weak oxidizing agent is dilute hydrogen peroxide.

Stage b) is preferably carried out in the presence of an alkaline agent, for example calcium hydroxide, sodium hydroxide, in the presence or in the absence of enzymes. In particular, stage b) is carried out with sulfur salts and alkaline treatments, such as Na₂S and Ca(OH)₂.

Preferably the acids used to acidify in step d) are selected from: hydrochloric, lactic, acetic, citric. Preferably it is acidified to pH between 1 and 3.

Preferably step f) for preparing an aqueous dispersion of fibrous collagen is carried out at a concentration between 1% and 5%. More preferably between 2% and 5%. In particular, it is preferred to subject the dispersion obtained in step f) to a mechanical homogenization process consisting in subjecting the dispersion to a shear force by passing it through a slit at high pressure, preferably at a minimum pressure of 50 atmospheres. Preferably between 50 atm and 100 atm.

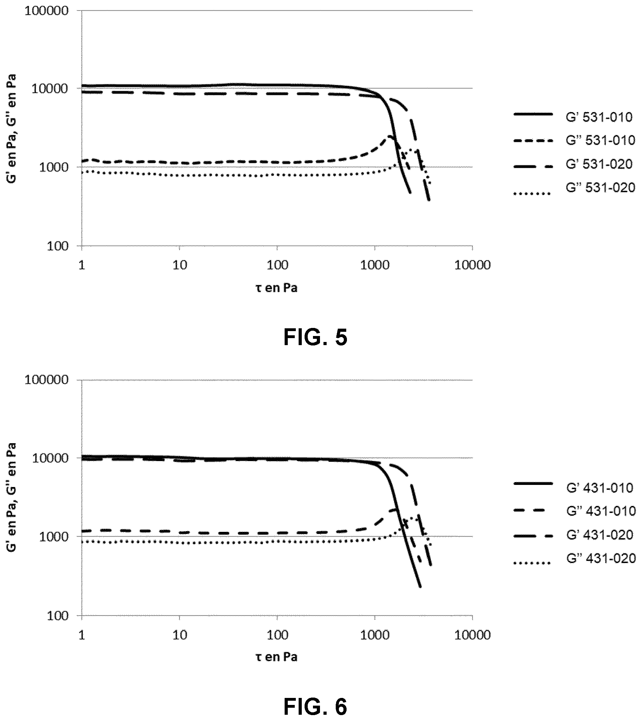

In Example 3 and Figures 5 and 6, it is shown that the homogenization process causes a change in the rheology of the ink of the invention. The homogenized mass is able to withstand more effort without losing its structure. The value of the elastic modulus (G') of the collagen mass remains constant when applying greater deformation forces than in the case of the non-homogenized mass.

The present invention also relates to the product obtained by the method of the invention and by all its embodiments described in the previous paragraphs.

The method of printing with the ink of the invention has different embodiments.

In one embodiment of the printing method, the ink is mixed with cells that are specifically selected from: astrocytes, embryonic cardiomyocytes, fetal cardiomyocytes, neonatal cardiomyocytes, cardiomyocytes, embryonic ventricular myocytes, corneal endothelial cells, corneal epithelial cells, iris pigment epithelial cells, retinal pigment epithelial cells, fetal dopamine neuronal cells, fetal neocortical neuronal cells, enteric neuronal cells, hepatocytes, adipose tissue mesenchymal stem cells, bone marrow mesenchymal stem cells, osteoblasts, chondrocytes, pancreatic cells, urothelial cells.

In another embodiment of the printing method, before printing, the ink is neutralized to a pH preferably between 7 and 8, in particular it is neutralized with salts or buffers selected from: phosphate buffer saline (1X and 10X PBS), Tricine, MOPS (3-(n-morpholino)propanesulfonic acid, HEPES (4-2(hydroxyethyl)piperazin-1-ylethanesulfonic acid), tris, sodium carbonate.

### EXAMPLES

The following examples are only illustrative of this invention, and should not be construed as limiting it.

### Example 1

To prepare the ink of the invention, 18-26 month old bovine skin is used which is subjected to a standard depilation process (e.g. with a sulfur salt such as Na₂S).

Next, the skin is divided and the dermis is extracted from where the collagen is obtained.

After several washes with water and bleaching with a mild oxidizing agent (for example, dilute hydrogen peroxide), the material is filed with calcium hydroxide under the time and temperature conditions that the material requires for its adequate maceration. The reagent remains are then washed again with water and acidified with concentrated HCl (33%). It is minced with the help of a grinder and diluted with water until obtaining a collagen dispersion in water at 2.9% in collagen. The resulting pH is 3.1.

### Example 2

The dispersion of Example 1, a mesh-shaped matrix (5X2.5 cm and 1.2 mm pore size) was printed with a 3D printer at a speed of 3 mm / s using a 24G nozzle needle extrusion head in a 10 cm Petri dish, by way of a scaffold as a substrate for cell culture.

Once the mesh was printed, it was immersed in 50 mM NaOH to cover the mesh to raise the pH and thus cause the collagen to coagulate. Once coagulated, it was washed with a phosphate buffer solution (PBS) at pH 7.4 in sufficient quantity to cover the mesh and it was subsequently immersed in culture medium enriched with antibiotics. It should be noted that this dispersion of the invention is printed and coagulated at room temperature and without adding crosslinking agents.

Murine embryonic cell fibroblasts were grown on the mesh. A total of 300,000 cells were resuspended.

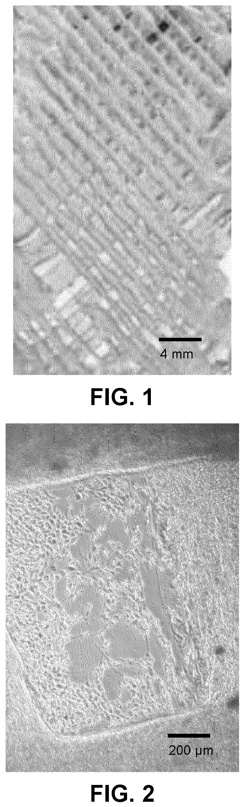

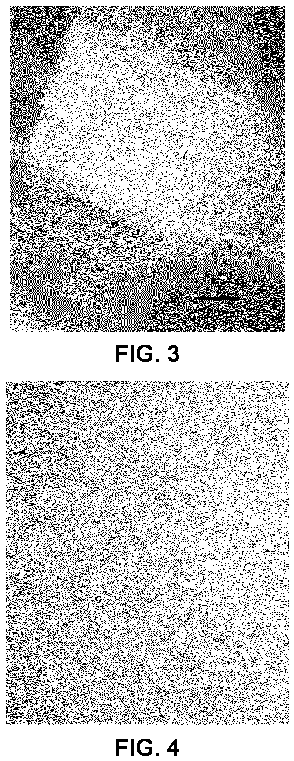

Figure 2 shows the culture of the meshes with fibroblasts obtained after 2 days. In Figure 3 the culture on the fifth day is shown. The culture plate is at 100% confluence and tactisms and directed growth of the cells with the material begin to be observed. Figure 4 shows the culture on the sixth day of development. Some cells grow in the direction of the collagen fibers in the mesh.

### Example 3 ink Rheology

For this, a controlled stress rheometer "Modulate Advanced Rheometer System" MARS 40 (Thermo Haake) equipped with a 20mm plate-plate rotor was used. An oscillatory stress sweep test was carried out in a stress range ( 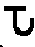 ) 1 Pa to 15000Pa at a fixed frequency of 1 Hz, maintaining a 0.8 mm gap between plates at a measurement temperature of 15.0 ° C. Prior to the test, the sample was allowed to temper for 10 minutes between the plates once the 0.8mm gap had been reached, to ensure the measurement temperature.

Stress sweep at a fixed frequency makes it possible to identify the zone of linear viscoelasticity.

Oscillatory measurements in the zone of linear viscoelasticity are of great importance because the response of the material will be only dependent on its structure and independent of rheological parameters such as stress or deformation.

During the test, the value of G' and G" (ordinate axis) is represented as a function of the stress (abscissa axis). The crossover, LVR and flow limit values correspond to the stress, that is, the abscissa axis.

The elastic or storage modulus (G') is associated with the energy stored in the material while the viscous or loss modulus (G") is associated with the energy dissipated by the material.

The crossover is the point where G' and G" are equal. It coincides with the value of the oscillatory shear stress where the curves of G' and G" intersect, and therefore it is the stress value from which the mass stops behaving as an elastic material and begins to behave as a viscous fluid.

The crossover values of the homogenized masses are approximately 40% higher than that of the diluted one, which implies that the mass maintains its elastic behavior at higher stresses and, therefore, is able to withstand more stress without losing its structure.

The stress sweep allows determining the yield point of the material. The value of the elastic modulus remains constant while the material does not flow (solid behavior), but its value decreases when the material begins to flow. The value of the elastic modulus at which the material begins to flow is the Flow Limit and indicates the point from which the mass begins to undergo permanent deformation. Up to that point, the deformation suffered by the mass was recoverable, and therefore the value of G' and G" was constant, but from the flow limit the mass begins to lose its elastic component and the viscous component increases, until in the crossover they become equal and begins to behave like a viscous fluid. Figures 5 and 6 show the stress sweep graphs for samples 531-010, 531-020 and 431-010, 431-020.

Samples 531-010 and 431-010 are equivalent to Example 1 but at collagen concentrations of 4.8 ± 0.1%. In the case of samples H (431-020 and 531-020) they are the same dispersions, but after having been subjected to a high pressure homogenization process in an industrial homogenizer that basically consists of passing the mass through a slit with help of a piston and at a pressure between 50 and 300 atmospheres.

The diluted and homogenized masses 531-020 and 431-020 have a higher flow limit than the diluted non-homogenized masses 531-010 and 431-010, and therefore can be subjected to a greater stress keeping their elastic component intact and, ultimately, its structural properties.

**Table 1 Rheology data of the inks 531-010, 431-010, 531-020 and 431-020 of the invention after performing a stress sweep at a frequency of 1 Hz**

| Sample | G' (Pa)* | G" (Pa)* | Crossover (Pa) | Flow limit (Pa) | Viscosity (illMcP) |
|---|---|---|---|---|---|
| 531-010 | 9558 | 1049 | 1654 | 1025 | 23.3 |
| 431-010 | 10292 | 1149 | 1706 | 1129 | 28.5 |
| 531-020 | 8218 | 742 | 2682 | 1453 | 6.5 |
| 431-020 | 9661 | 860 | 2941 | 1691 | 5.3 |

| | | | | | |
|---|---|---|---|---|---|
| * Corresponds to the value of G' and G" during the first 100 seconds of analysis, ensuring that it is in the linear phase of the curve. | | | | | |

A quick way to quantify the degree of degradation and hydrolysis of native collagen is to determine the soluble fraction at 10% ammonium sulfate after centrifugation at 18,000 rpm. Subsequently, the collagen protein content in said fraction is quantified with the Biuret method and the result is expressed as a percentage with respect to the percentage of collagen, previously quantified using the Biuret method. Said fraction especially appears in deteriorated collagens.

Subsequently, the collagen protein content in said fraction is quantified with the Biuret method and the result is expressed as a percentage (Table 2).

**Table 2. % solids, and soluble fraction.**

| Sample | % solids | % soluble fraction |
|---|---|---|
| (531-010) Without homogenizing | 4.8 | ND |
| (531-020) After homogenizing | 4.8 | ND |

| | | |
|---|---|---|
| • ND: Not detected | | |

The viscosity of the dispersion of the invention was measured.

The viscosity of the inks of the invention was determined using the Brookfield viscometer model DV2T HBT (Brookfield Ametek). Viscosity is calculated from the measured torque based on selected spindle and rotational speed.

The spindles used for this method are those of inverted T. (Helipath with T-Bar Spindle). The following are the viscosity ranges measured by each spindle for that viscometer model.

| T-Bar Spindle | Spindle code | SMC | Viscosity (cP) |
|---|---|---|---|
| T-A | 91 | 20 | 16.000 - 16.000.000 |
| T-B | 92 | 40 | 32.000 - 32.000.000 |
| T-C | 93 | 100 | 80.000 - 80.000.000 |
| T-D | 94 | 200 | 160.000 - 160.000.000 |
| T-E | 95 | 500 | 400.000 - 400.000.000 |
| T-F | 96 | 1000 | 800.000 - 800.000.000 |

| | | | |
|---|---|---|---|
| * SCM (Spindle Multiplier Constant) is a characteristic constant of each spindle used to convert the torque measurement into viscosity. | | | |

Other spindles are used for lower viscosity ranges, masses with a concentration equal to or less than 2% collagen:

| Spindle | Viscosity range (cP) |
|---|---|
| HB-1 | 2.096 - 20.960 |
| HB-2 | 8.880 - 88.800 |
| HB-3 | 42.800 - 428.000 |
| HB-4 | 434.400 - 4.344.000 |
| HB-5 | 170.400 - 1.704.000 |

The equipment is configured in such a way that it is necessary to indicate the spindle that is used and the rotation speed and it itself makes the conversion to viscosity in cP.

For this, the mass was placed in a 250 ml beaker. The temperature of the mass was in a range comprised between 18°C and 22°C. The corresponding spindle was used depending on the expected viscosity of the mass. Thus, 94 was used for the homogenized masses (531-020), while 95 was used for the non-homogenized ones (531-010). The rotation speed was 1.0 RPM and 250 measurements were taken during 4 minutes .

The viscosity measurement was notably reduced after homogenization, resulting in 23.3 and 6.5 McP for 531-010 and 531-020 respectively (Table 1). This reduction in viscosity greatly facilitates the printability of the ink and reduces the pressure required to print the ink with the printer.

### Example 4. Effect of pH on ink

In this case, the behavior of the ink of the invention and the neutralized ink of the invention has been compared. It is important to maintain the structure and firmness of the material after neutralizing to a physiological pH and to ensure that the structure and shape of the 3D construct to be printed will remain firm enough and remain stable under the physiological conditions required for use in biomedical applications in which they are to be used as a support for cell growth. As derived from the following table (Table 3), despite the reduction in elastic modulus, the G' value of the neutralized material (531-010 Neutral and 531-020 Neutral) remains high. Therefore, said material remains firm enough over time after neutralizing to physiological pH with different bases and placing it in a medium similar to cell culture media such as: PBS, Hank's solution enriched with Calcium (HBSS- Ca) or equivalent.

A rheological analysis was carried out by means of a frequency sweep that consisted of an oscillatory test at a fixed stress of 100Pa, making a frequency sweep in a range of 0.01 Hz to 100 Hz and with a gap between plates of 0.8 mm, being the measurement temperature of 15 ° C. In the following table the values obtained for a frequency of 0.1 Hz are represented, wherein it can be observed that the stability of the material of the invention of Example 3 at a collagen concentration of 4.8%, after neutralizing it to physiological pH with NaOH (0.05M ) and balancing it in PBS, it maintains a very high elastic modulus (G'):

**Table 3 Rheological parameters of the ink of the invention before and after neutralization to neutral pH. Values at a frequency of 0.1Hz.**

| Sample | G' (Pa) | G" (Pa) |
|---|---|---|
| 531-010 | 7800 | 800 |
| 531-010 Neutral | 3400 | 400 |
| 531-020 | 6600 | 610 |
| 531-020 Neutral | 3400 | 370 |

In the same way, the stability over time of the neutralized material was determined. For this, another batch (batch 6) prepared as the gel of the invention of Example 3 was neutralized with NH₄OH (0.25%) or with NaOH (0.05M) and adjusted to physiological pH with HBSS-Ca. A sample quantity of approximately 0.3 g was taken and neutralizing agent was added to cover it.

In the following table it can be seen that the material, once neutralized with NaOH and after being washed and equilibrated with PBS at physiological pH, maintains, despite an initial drop, a high and stable elastic modulus over time, which confirms the maintenance of its structure under physiological conditions equivalent to those used for cell culture (37°C and pH 7.4). A similar observation is obtained with other bases such as ammonium hydroxide and other balancing media such as HBSS-Ca, which confirms the maintenance of the stable construct shape in the usual culture media:

**Table 4 Evolution of the storage modulus or elastic modulus (G') of the sample 231-020 neutralized over time. Values at a frequency of 0.1Hz.**

| G' (Pa) | Day | Base |
|---|---|---|
| 4675 | 0 | NaOH |
| 3242 | 7 | NaOH |
| 3038 | 14 | NaOH |

### Example 5. Printing with the ink in the Example 3

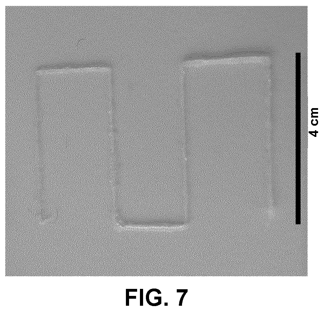

A printing was made at an extrusion speed of 2 mm / second adjusting the pressure of the equipment accordingly (550kPa) and using a 22G nozzle needle extrusion head. Despite being an ink with insoluble collagen fibers, continuity in the construct was appreciated, as seen in Figure 7.

### Example 6. Printability of the ink of the invention.

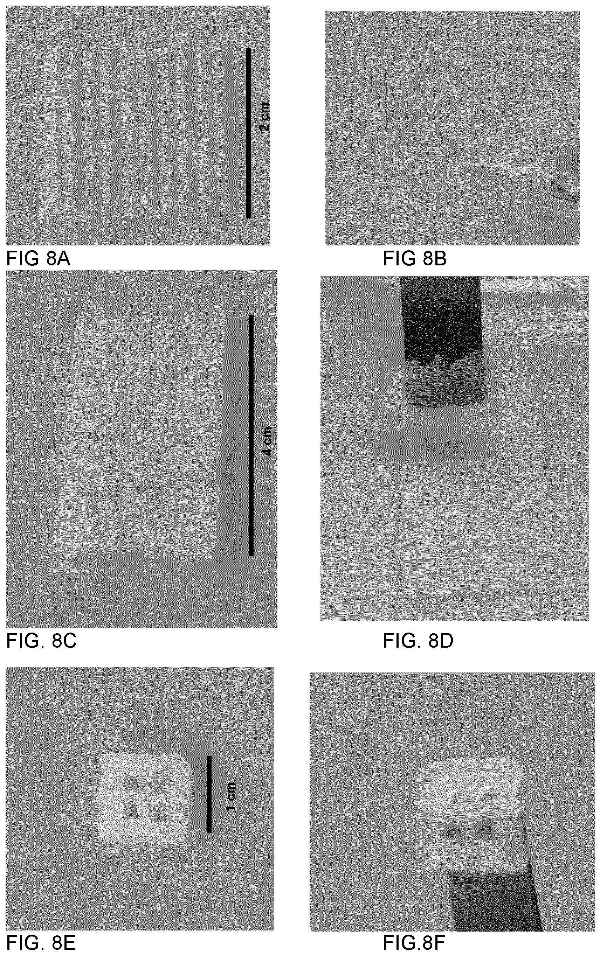

Figures 8A-8F show examples of structures obtained with the ink of Example 3 at an extrusion speed of 5 mm / second by adjusting the pressure of the 3D printer accordingly. The continuity in the constructs is appreciated.

Figures 8E-F correspond to multi-layer 3D constructs.

Figures 8B, 8D, 8F, correspond to the same structures on the left, once neutralized with 50 mM NaOH and adjusted to pH 7.4, physiological pH, with PBS, pH suitable for optimal cell proliferation. In all cases, the integrity of the structure is maintained and its manipulation is allowed for applications such as those previously indicated.

When said ink of the invention was prepared at concentrations less than 1%, a great decrease in the firmness of the construct was observed, with viscosity values of 6,000 cP, for an ink with 1% collagen. Therefore, these conditions are not optimal in the event that the construct or matrix is to be neutralized, but the ink can be useful when the printed structure does not need neutralization.

It happened in a similar way if the pH of the ink was excessively reduced, in which the elasticity of the 531-020 ink decreased by lowering the value of G' to 3900 Pa and the viscosity to 3.14 McP when the pH of the ink was 0.6. After printing, the construct lost its firmness during the neutralization process.

The use of hydrochloric, lactic or acetic acid during the acidification of step d) of the production process gave rise to perfectly printable inks that maintained the integrity of the constructs after neutralization at the aforementioned pH and concentration values.

### Example 7. Morphological analysis

A morphological analysis was carried out by means of dynamic image analysis of the inks 531-010 and 531-020 of Example 3. For this, the ink was diluted approximately 1% in deionized water, and adjusted to an approximate pH of 1.5 with 1M HCl. The diluted sample was vortexed for 10 minutes at 2500 rpm and diluted 1/20 with deionized water.

Measurements were performed on a Sympatec QICPIC / Lixell particle analyzer with a continuous flow tray (0.5 mm wide) in a particle size measurement range of 4 - 2888 µm. Due to the great heterogeneity of particle distribution, typical of collagen suspensions, at least 3 individual measurements of two subsamples were performed.

In total, between 1 and 3 million particles were detected per sample.

An evaluation was made based on the fiber length and diameter distribution. The proportion of fibers of a certain length or width in the total mass of collagen fibers in the sample was calculated. Tables 5 and 6 indicate the length and diameter (in µm) for the percentages of 10, 16, 50, 84 and 90% of the cumulative distribution of collagen fibers in the total mass, calculated from the mean values.

**Table 5. Fiber lengths (µm) corresponding to the accumulated percentages of 10, 16, 50, 84 and 90% of total mass of collagen fibers in the inks of the invention 531-010 and 531-020**

| | 10% | 16% | 50% | 84% | 90% |
|---|---|---|---|---|---|
| 531-010 | 244 | 336 | 834 | 1775 | 2291 |
| 531-020 | 99 | 135 | 383 | 989 | 1235 |

**Table 6. Fiber diameters (µm) corresponding to the accumulated percentages of 10, 16, 50, 84 and 90% of total mass of collagen fibers in the inks of the invention 531-010 and 531-020.**

| | 10% | 16% | 50% | 84% | 90% |
|---|---|---|---|---|---|
| 531-010 | 9,3 | 11,6 | 19,0 | 27,0 | 30,4 |
| 531-020 | 10,2 | 12,3 | 19,2 | 27,6 | 30,8 |

As seen in Table 5, sample 9D shows a broader fiber length distribution. The median value is 838 µm. This means that 50% of the total collagen fiber mass in the sample is provided by fibers with lengths greater than 838 µm. 84% of the collagen mass is provided by fibers with a fiber length of more than 336 µm. However, in 531-020, the proportion of smaller fibers is much higher. Its median value of 383 µm is significantly lower than for sample 531-010. This means that half the collagen mass of sample 531-020 consists of fibers less than 383 µm in length, compared to 838 µm in the case of 531-010.

Therefore, with homogenization the size of the fibers is shortened and this facilitates the passage of the ink through the extrusion head and, ultimately, its printability, reducing the pressure necessary to be able to print it with commercial printers.

The cumulative distribution of the fiber diameters of samples 531-010 and 531-020 (Table 6) are almost identical with a median value of 19 µm, with 80% of fibers in a diameter range between 10 µm and 31 µm,

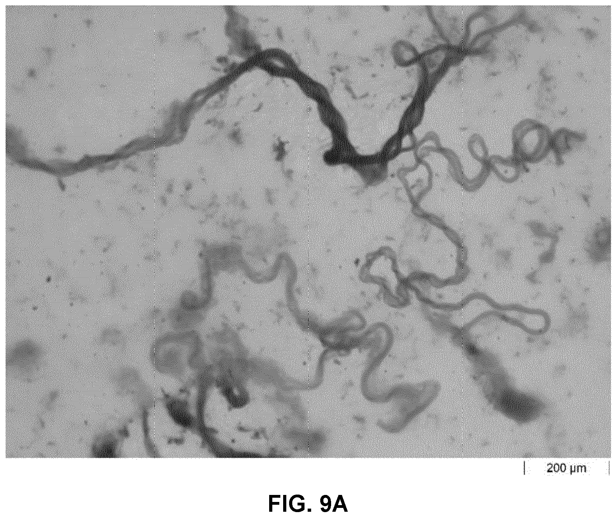

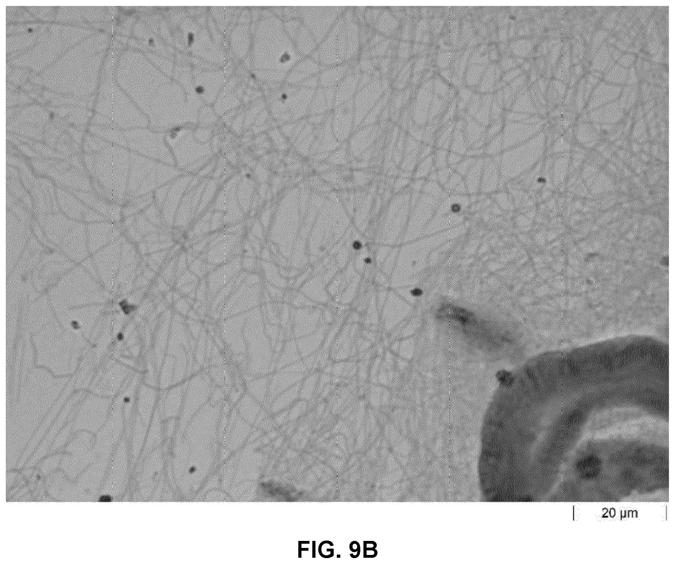

Figures 9A and 9B show images by optical microscopy of individual particles of the ink of the invention 531-010 diluted to 0.1% and stained with Sirius Red. Said figures allow having a visual image of the fibers present in the ink of the invention, wherein the broad distribution of fiber lengths and diameters is clearly observed.

### Example 8

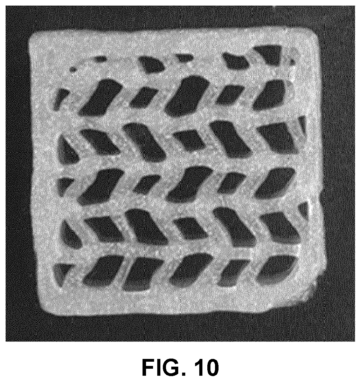

Prior to printing, the acidic ink of Example 1 was neutralized but with a concentration of 5%, mixing it with 1M tris(hydroxymethyl)aminomethane (Tris) adjusted to pH 7.4 in a 3: 2 (V: V) ratio to obtain a concentration of 3%. The ratio could be varied to achieve more diluted inks. Likewise, half of the buffer can be replaced by culture medium such as DMEM (Dulbecco's Modification of Eagle's Medium). For mixing, a syringe was filled with 3 ml of collagen and another with 2 ml of Tris. With the help of a connector, the two syringes were connected and the content was passed from one to the other, repeating this last step 40 times, thus allowing the content to be homogeneously mixed. Subsequently, it was printed adjusting the pressure to 70kPa at an extrusion speed of 5mm / s using a 20G nozzle needle extrusion head. The impression made is shown in Figure 10.

### Example 9. Cell encapsulation in neutral collagen ink

The quantities suggested in this example on cell density are based on studies with murine fibroblast cells. The method used in this example for mixing the different components is carried out in a similar way to that of example 8 of neutralized ink.

The collagen mass was obtained at a neutral pH as mentioned in example 1. In this case, from the quantities calculated for the collagen mass mixture at acid pH with the corresponding buffer, the necessary quantity was calculated for subsequent mixing with cells. Forthis, three parts of collagen, one of buffer and one of DMEM (Dulbecco's Modification of Eagle's Medium) culture medium were mixed with the cells. On this occasion, the acidic collagen mass was mixed with the corresponding buffer 40 times (as mentioned in the previous example of neutralized ink) and then, it was mixed in the same way with the culture medium containing the cells, but this time a total of 20 times.

To obtain the culture medium with the cells, a normal cell harvesting protocol was carried out to obtain them in suspension with an approximate density of 200,000 cells 1 ml.

Once all the components had been mixed, it was bioprinted on plates prepared for cell culture. Once the constructs were bioprinted, enriched DMEM was added to cover the entire scaffold or construct. Cell viability tests (Live / Dead Assay) were performed 5 days after bioprinting the cells. To complement the study, the scaffolds (support) were digested using a combined trypsin-EDTA and collagenase treatment to obtain the cells, both those on the surface and those embedded in the ink (first incubation of 20 minutes with Trypsin-EDTA 0.05%; followed by a second one with collagenase 500U / ml until the scaffold has completely been dissolved). The results showed cell viability greater than 90%.

## Claims

1. Collagen ink for 3D printing comprising a dispersion of native collagen fibers in an acid medium at a concentration between 0.1% and 10% with a pH between 0.1 and 5, which has a viscosity between 10 cP and 50 McP (Brookfield method at a temperature between 18°C and 22°C) and wherein the fiber dispersion comprises more than 90% of fibers whose length, measured at a pH between 1 and 2, is in a range from 1µm to 2500µm and its diameter in a range from 0.1 µm to 150µm.

2. Collagen ink for 3D printing according to claim 1 **characterized in that** at least 90% of the mass of collagen fibers of the dispersion comprises fibers whose length is in a range from 50 µm to 2500 µm.

3. Collagen ink for 3D printing according to claim 1 y 2 **characterized in that** 75% of the mass of collagen fibers of the dispersion comprises fibers whose length is in a range of 50µm to 1000µm.

4. Collagen ink for 3D printing according to claim 1 y 2 **characterized in that** 50% of the mass of collagen fibers in the dispersion comprises fibers whose length is in a range between 100 µm and 500 µm.

5. Collagen ink for 3D printing according to one of the claims 1 to 4 **characterized in that** 80% of the mass of collagen fibers comprises fibers whose diameter is preferably between 5 µm and 35 µm.

6. Collagen ink for 3D printing according to one of the claims 1 to 5 **characterized in that** the concentration is between 1% and 5%.

7. Collagen ink for 3D printing according to one of the claims 1 to 6 **characterized in that** the pH is between pH 1 and 3.

8. Collagen ink for 3D printing according to one of the claims 1 to 7 **characterized in that** the viscosity is between 2 McP and 15 McP.

9. Hydrogel comprising the ink according to any of claims 1 to 8.

10. Method of obtaining the ink according to claims 1 to 8 from a collagen-containing tissue comprising the steps of:
a) washing, and chopping a collagen-containing tissue;
b) chemically macerating the chopped tissue in a controlled manner;
c) washing the product obtained from step b) with water;
d) adjusting the pH of the side product obtained in c), to values between 0.5 and 5 to swell the side product of step c):
e) mechanically mincing the product of step d);
f) dispersing in water to a concentration preferably between 0.1% and 10%.

11. Method according to claim 10 **characterized in that** the collagen-containing tissue is connective tissue.

12. Method according to claim 11 **characterized in that** the connective tissue is dermis tissue of the layer called corium.

13. Method according to any one of claims 10 to 11, **characterized in that** the connective tissue is of bovine origin, with an age between 1 and 3 years.

14. Method according to any of the claims 10 a 13 **characterized in that** step c) is carried out in the presence of an alkaline agent.

15. Method according to any of the claims 10 to 14 **characterized in that** in step d) it is acidified between 1 and 3.

16. Method according to any of claims 10 to 15, **characterized in that** there is a stage of homogenization of the precipitate obtained in stage d) prior to stage e).

17. Method according to any of claims 10 to 16, **characterized in that** the aqueous dispersion of fibrous collagen is carried out at a concentration that is between 1% and 5%.

18. Use of the ink according to any one of claims 1 to 8 in printing 3D structures.

19. Structures comprising the ink according to any one of claims 1 to 8.

20. Method of printing with the ink described in any of claims 1 to 8, **characterized in that** it comprises the step of mixing the ink described in any of claims 1 to 8 with cells before printing.

21. Method according to claim 20 **characterized in that** the cells are selected from: astrocytes, embryonic cardiomyocytes, fetal cardiomyocytes, neonatal cardiomyocytes, cardiomyocytes, embryonic ventricular myocytes, corneal endothelial cells, corneal epithelial cells, iris pigment epithelial cells, retinal pigment epithelial cells, fetal dopamine neuronal cells, fetal neocortical neuronal cells, enteric neuronal cells, hepatocytes, adipose tissue mesenchymal stem cells, bone marrow mesenchymal stem cells, osteoblasts, chondrocytes, pancreatic cells, urothelial cells.

22. Method of printing the ink described in any of claims 1 to 8, **characterized in that** it comprises the steps of:
neutralizing the ink to a physiological pH, preferably between 7 and 8;
printing the structure.

23. Method according to claim 22 **characterized in that** ink mixes with cells.

24. Method according to claim 22 **characterized in that** the neutralization is carried out with salts or buffers selected from: phosphate buffer saline (PBS 1X and 10X), Tricine, MOPS, HEPES, Tris, sodium carbonate.
